# EUROPEAN PATENT APPLICATION

(11) **EP 2 380 607 A2**
(43) Date of publication of application: **26.10.2011**
(21) Application number: 11250308.1
(22) Date of filing: 15.03.2011
(51) Int. Cl.: A61L 33/06

(54) **Mitigating thrombus formation on medical devices by influencing ph microenvironment near the surface**

(30) Priority: 16.03.2010 US 724472
(71) Applicant: Confluent Surgical Inc., Waltham, Massachusetts 02451 (US)
(72) Inventor: Ohri, Rachit, Framingham, MA 01701 (US); Blaskovich, Philip, Salem, MA 01970 (US); Bennett, Steven L., Cheshire, CT 06410 (US); Tramontano, Valentino, Brockton, MA 02301 (US)
(74) Representative: Soames, Candida Jane

(57) **Abstract**

The present disclosure provides treatments of medical devices which inhibit thrombus formation. At least a portion of a substrate of a medical device includes a surface possessing a functionality and / or surface charge adapted to modulate the pH of the surface of the medical device, as well as the pH microenvironment near the surface of a medical device.

## Description

### TECHNICAL FIELD

The present disclosure relates to medical devices having anti-thrombogenic properties. More particularly, the present disclosure relates to coatings or surface treatments for medical devices which regulate the pH of the local environment around the device in a range that is not conducive to thrombus formation.

### BACKGROUND OF RELATED ART

Thrombosis, the formation of a clot or thrombus in the cardiovascular system from the constituents of blood, may lead to negative clinical outcomes and is a potentially life threatening condition. Medical devices which have direct contact with blood flow, such as stents, vascular grafts and defibrillators, have a tendency to promote localized thrombosis. For example, thrombus formation on drug eluting stents may lead to late stent thrombosis.

Thrombus formation may be inhibited by a variety of treatment methods. For example, surface treatments based on the binding of coagulation inhibitors such as platelet aggregation inhibitors, plasminogen activators, fibrinogen, and/or heparin have been used on implants. These treatments, however, may include side effects, require blood testing to monitor anticoagulation levels, and are subject to degradation over time.

Improved materials and treatment methods for implants, which avoid thrombus formation, remain desirable.

### SUMMARY

The present disclosure provides medical devices and methods for producing such devices. In embodiments, a medical device of the present disclosure includes at least one substrate possessing a surface; and a functionality on at least a portion of the surface, wherein the functionality modulates a pH of a microenvironment near the surface of the medical device.

In other embodiments, a medical device of the present disclosure includes at least one substrate possessing a surface; and a functionality on at least a portion of the surface, wherein the functionality modulates a pH of a microenvironment near the surface of the medical device to prevent thrombus formation.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a side view of a surface of a medical device of the present disclosure, depicting the microenvironment pH near the surface of the medical device;

FIG. 2 is a perspective view of an example of an expanded stent which may be treated in accordance with the present disclosure to alter the microenvironment pH near the surface of the stent;

FIG. 3 illustrates a cross sectional view of a bifurcated graft inserted within an aortic aneurysm, the graft having been treated in accordance with the present disclosure to alter the microenvironment pH near the surface of the graft; and

FIG. 4 illustrates an internal defibrillator utilizing paddles that are controlled by two separate handles that can be separated or locked at a certain desired distance between the electrodes so as to operate as a single unit.

### DETAILED DESCRIPTION

In accordance with the present disclosure, medical devices are provided which may be formed from, or treated with, materials which modulate the pH of the surface of the medical device, as well as the pH of the microenvironment near the surface of the medical device, in order to prevent thrombus formation. As used herein, "microenvironment" includes the area near the surface of a device which may exhibit a pH that is close to, but different than, the bulk pH of the environment in which the device is placed. The present disclosure may be used to prevent thrombosis-related failure of medical devices, as well as to prevent negative clinical outcomes for patients who develop thrombosis due to the presence of implanted medical devices.

The pH of a surface of a medical device, as well as the pH of the microenvironment near the surface of the device, may be adjusted so as to prevent thrombus formation by including a material possessing the necessary features to create a microenvironmental pH that is different than the bulk pH of the device or the bulk pH of blood in the area. The affect of pH on thrombosis has been studied, for example, by Thomas et al., "High Molecular Weight Kininogen Adsorption on Hemodialysis Membranes: Influence of pH and Relationship with Contact Phase Activation of Blood Plasma-influence of Pre-treatment with Poly(ethyleneimine)," International Journal of Artificial Organs, Vol. 23, pp. 20-26 (2000), the entire disclosure of which is incorporated by reference herein.

In embodiments, a surface of a medical device may possess a functionality capable of modulating the pH of the material forming the surface, as well as the pH of the microenvironment near the surface of the device. The material may attract positive or negative ions from the surrounding environment to create a different pH in the microenvironment around the medical device. The ability to control the microenvironmental pH allows for the control of factors which may lead to thrombosis, such as, for example, enzymatic activity during the coagulation process.

In scenarios where blood clot formation is favored at a more basic pH, the surface of the medical device, as well as the pH of the microenvironment near the surface of the device, may possess a slightly more acidic pH to avoid or mitigate thrombus formation. For instance, an acidic pH may decrease the efficacy of Factor VII (e.g., in the extrinsic pathway of the clotting cascade) in initiating blood coagulation. See, for example, Bladbjerg et al., "Activity of Recombinant Factor VIIa under Different Conditions In Vitro: Effect of Temperature, pH, and Haemodilution," Blood Coagulation and Fibrinolysis, Vol. 19, No. 5, pp. 369-374 (2008), the entire disclosure of which is incorporated by reference herein. Thus, a device with a more acidic pH at its surface or in the microenvironment adjacent to the surface may aid in the prevention or delay the formation of a thrombus at or near the surface of the device.

In other scenarios where blood clot formation is favored at an acidic pH, the surface of the medical device, as well as the pH of the microenvironment near the surface of the device, may possess a slightly more basic pH to avoid or mitigate thrombus formation. Thus, a device with a more basic pH at its surface or in the microenvironment adjacent such surface may aid in the prevention or delay of such a process, including its impact on thrombus formation.

Alternatively, surface charge may significantly impact the kinetics of thrombus formation on and near the surface of a medical device. For instance, blood components may coagulate upon contact with negatively charged surfaces (e.g., in the intrinsic pathway of the clotting cascade). See, for example, Norris et al., "Blood Coagulation," Best Pract Res Clin Obstet Gynaecol., 2003 Jun;17(3):369-83, the entire disclosure of which is incorporated by reference herein. Thus, medical device surfaces with a positive charge will, in these scenarios, retard thrombus formation.

In embodiments, the pH of a surface of a medical device and the microenvironment near the surface may be adjusted by imparting a charge thereto. A charge may be imparted to the surface of a medical device by admixing a material capable of imparting a charge to the material utilized to form the medical device, in embodiments a polymer, or by applying a coating including a material capable of imparting a charge to a surface of the medical device. In other embodiments, the medical device may have surface functional groups capable of modulating the pH of the materials at or near the surface of the device. The ability to modulate local pH and/or to have pH buffering capacity in the microenvironment near the surface will allow for the prevention or retardation of the rate of thrombus formation. These modifications may affect both the local pH, as well as the pH buffering capacity of the microenvironment near the surface of the device.

Materials may be formed either through covalent, ionic or hydrophobic bonds. Physical (non-covalent) crosslinks may result from complexation, hydrogen bonding, desolvation, Van der Waals interactions, ionic bonding, combinations thereof, and the like. Chemical (covalent) crosslinking may be accomplished by any of a number of mechanisms, including free radical polymerization, condensation polymerization, anionic or cationic polymerization, step growth polymerization, electrophile-nucleophile reactions, combinations thereof, and the like.

Certain properties of the material can be useful, including adhesion to a variety of tissues, mechanical strength for use in medical devices, and/or toughness to resist destruction after placement. Synthetic materials that are readily sterilized and avoid the dangers of disease transmission involved in the use of natural materials may thus be used. The material may have anti-thrombotic properties and be unreactive with blood or other body fluids. Generally, the materials should also be selected on the basis of exhibited biocompatibility and lack of toxicity.

By forming the medical device with materials capable of influencing pH, the pH of a surface of a medical device, as well as the pH of the microenvironment near the surface of the medical device, may be adjusted so that the medical device is anti-thrombotic. Thus, as noted above, in some scenarios where blood clot formation is favored at an acidic pH, the surface of the medical device, as well as the pH of the microenvironment near the surface of the device, may possess a slightly more basic pH to avoid or retard thrombus formation. In other scenarios where blood clot formation is favored at a more basic pH, the surface of the medical device, as well as the pH of the microenvironment near the surface of the device, may possess a slightly more acidic pH to avoid or retard thrombus formation.

In embodiments, it may be desirable to adjust the pH microenvironment of the entire surface of the medical device, and in other embodiments, it may be desirable to adjust the pH microenvironment of the tissue-facing and/or non-tissue facing surfaces of the medical device. In yet other embodiments, it may be desirable to adjust the pH microenvironment of the blood or bodily fluid contacting surfaces of the medical device.

In embodiments, the pH of a surface of a medical device, as well as the pH of the microenvironment near the surface of the medical device, may be altered utilizing a charged polymer to form the device, or by admixing a material capable of imparting a charge to the material utilized to form the medical device, in embodiments a polymer. Methods for forming such polymers or combining materials with polymeric materials are within the purview of those skilled in the art and include blending, mixing, stirring, copolymerizing, combinations thereof, and the like.

In other embodiments, the pH of the surface of a medical device, as well as the pH of the microenvironment near the surface of the medical device, may be altered by applying a coating including a material capable of imparting a charge to a surface of the medical device that may be in contact with blood, to aid in controlling the local pH microenvironment. Such a coating should not affect the pH of the blood, but should be applied to at least a portion of the surface of the medical device in contact with blood.

Methods for applying a coating are within the purview of those skilled in the art and include, but are not limited to, dipping, spraying, plasma deposition, combinations thereof, and the like. In yet other embodiments, the surface of a medical device, as well as the pH of the microenvironment near the surface of the medical device, may be adjusted by applying a coating or a film, e.g., of a cured coating solution, to the surface of the medical device.

Examples of charged polymers that may be utilized in forming a medical device or a coating to be applied thereto include, but are not limited to, 2-hydroxyethyl methacrylate (HEMA), 2-acrylamido-2-methylpropane sulfonic acid (AAMPS), 3-methacryloylaminopropyl-trimethyl ammonium chloride (MAPTAC), N,N-diallyl-N,N-dimethyl ammonium chloride (DADMAC), combinations thereof, and the like.

Thus, for example, where the polymer is based upon MAPTAC, it will possess a positive charge due to the presence of a quaternary ammonium group, which remains cationic at all pH values. In embodiments, a copolymer of MAPTAC and HEMA may be utilized which attracts negatively charged low-molecular weight species such as hydroxyl ions and repels hydrogen ions. Such a copolymer may possess MAPTAC in an amount from about 0.1 percent by weight to about 10 percent by weight of the copolymer, and HEMA in an amount from about 90 percent by weight to about 99.9 percent by weight of the copolymer. In other embodiments, such a copolymer may possess MAPTAC in amounts from about 0.2 percent by weight to about 5 percent by weight of the copolymer, with HEMA present from about 95 percent by weight to about 99.8 percent by weight of the copolymer.

Alternatively, where a charged polymer is based upon AAMPS, it will possess a negative charge due to the presence of its sulfonate group, which remains ionized even in highly acidic conditions. In embodiments, a copolymer of AAMPS in HEMA may be utilized which will thus attract hydrogen ions (or protons). Such a copolymer may possess AAMPS in an amount from about 0.1 percent by weight to about 10 percent by weight of the copolymer, with the HEMA present in an amount from about 90 percent by weight to about 99.9 percent by weight of the copolymer, in embodiments the AAMPS may be present in an amount from about 0.2 percent by weight to about 5 percent by weight of the copolymer, with the HEMA present in an amount from about 95 percent by weight to about 99.8 percent by weight of the copolymer.

In other embodiments, a charged polymer may be formed with hydroxypropyl methylcellulose, acrylic acid copolymers, maleic acid copolymers, methacrylic acid copolymers, and the like, including a copolymer of methacrylic acid with ethyl acrylate, combinations thereof, and the like. Copolymers of methacrylic acid with ethyl acrylate include those commercially available under the EUDRAGIT® name from Rohm Pharma Polymers (Piscataway, NJ). In embodiments, these polymers may be charged by incorporation of an acid therein. Suitable acids which may be included in such copolymers may include, for example, citric acid, fumaric acid, succinic acid, malic acid, combinations thereof, and the like. Where an acid is added to a polymer to form a charged polymer, the acid may be added in an amount from about 0.1 percent by weight to about 10 percent by weight of the copolymer, in embodiments from about 0.5 percent by weight to about 5 percent by weight of the copolymer.

Other polymers may also be utilized. As noted above, in embodiments, a polymer may possess functional groups capable of altering the pH of a surface of a medical device, as well as the pH of the microenvironment near the surface of the medical device. For example, in embodiments, one could utilize the reaction of succinic anhydride with any hydroxyl or amine-functional polymer to generate a carboxylated polymer. Such polymers have an ability to affect the pH microenvironment when utilized to form a portion of a medical device or a coating thereon, as they are capable of neutralizing bases through neutralization with the carboxylic acid group to form the carboxylic acid anion. A summary of this reaction is provided below:

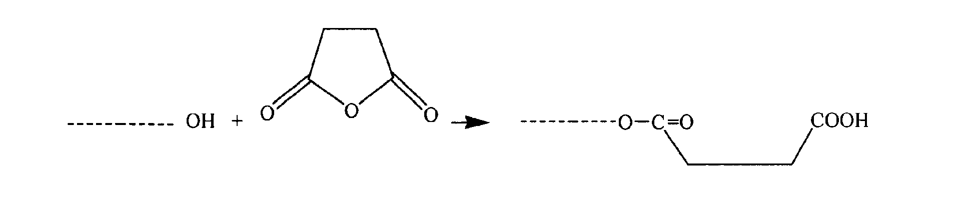

In other embodiments, one could use glycidyl methacrylate (GMA) in copolymers to provide pendant epoxy functionality. The epoxy group has the ability to absorb acids (protons) and undergo a ring opening reaction, thus becoming protonated. Thus, such a copolymer also has the ability to affect the pH of an aqueous microenvironment. The relevant chemical structure is provided below for the GMA monomer, which is the precursor to the GMA polymer.

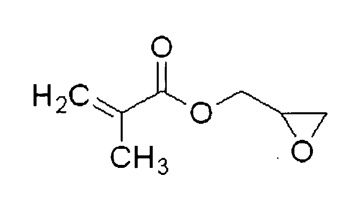

In yet other embodiments, acetoacetoxyethyl methacrylate (AAEM) copolymers may be utilized. AAEM copolymers can chelate a metal ion, in embodiments a divalent or multivalent ion, between its two carbonyl groups, which could then impart charge into the polymeric structure. Metal ions which could be chelated by such a copolymer include, but are not limited to, silver, cobalt, zinc, calcium, magnesium, platinum, tin, selenium, manganese, combinations thereof, and the like. In embodiments, an anionic (negative) charge may be created in a basic environment devoid of cations or metal ions. The relevant chemical structure is provided below for the AAEM monomer, which is the precursor to the AAEM polymer.

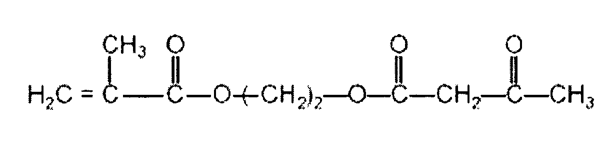

The formation of suitable copolymers is within the purview of those skilled in the art and may include the use of crosslinkers such as multi-functional acrylates or methacrylates, photoinitiators such as benzoin ethyl ethers, combinations thereof, and the like.

The local pH microenvironment of a surface of a medical device, as well as the pH of the microenvironment near the surface of the device, due to the presence of the charged polymer and/or functional group, may be from about 3 to about 11, in embodiments from about 5 to about 9. In some embodiments, the local pH microenvironment may be from about 6.0 to about 7.39 and in other embodiments from about 7.41 to about 8.5.

Methods for determining the pH microenvironment are within the purview of those skilled in the art and include, for example, amperometric and potentiometric microelectrodes, such as the ORION® microelectrodes by Thermo Fisher Scientific (Waltham, MA); optical and fluorescent pH sensors, including hollow fiber membranes micro probes; ion selective membranes; ion selective field effect transistors; two terminal micro sensors; metal oxide and conductometric pH-sensing devices; and confocal laser scanning microscopy (CLSM), a high resolution and non-invasive technique to monitor pH continuously and spatially resolved, as further disclosed by Agi, et al., "Fluorescence Monitoring of the Microenvironmental pH of Highly Charged Polymers," Journal of Polymer Science, Part A, Polymer Chemistry, pp. 2105-2110 (1997); Tatavarti, et al., "Microenvironmental pH Modulation Based Release Enhancement of a Weakly Basic Drug from Hydrophilic Matrices," Journal of Pharmaceutical Sciences, Vol. 95, No. 7, pp. 1459-1468 (2006); Liermann, et al. "Microenvironments of pH in Biofilms Grown on Dissolving Silicate Surfaces," Chemical Geology 171, pp. 1-16 (2000); Korostynska et al. "Review Paper: Materials and Techniques for In Vivo pH Monitoring," IEEE Sensors Journal, Vol. 8, No.1, pp. 20-28 (2008); Ruiz-Ederra, et al., "In Situ Fluorescence Measurement of Tear Film [Na+], [K+], [Cl-], and pH in Mice Shows Marked Hypertonicity in Aquaporin-5 Deficiency," Investigative Ophthalmology & Visual Science, Vol. 50, No. 5, pp. 2132-2138 (2009); Grant, et al., "A Sol-gel Based Fiber Optic Sensor for Local Blood pH Measurements," Sensors and Actuators, B 45, pp. 35-42 (1997); and Korostynska et al. "Review on State-of-the-art in Polymer Based pH Sensors," Sensors, Vol. 7, pp. 3027-3042 (2007), the entire disclosures of each of which are incorporated by reference herein.

Utilizing the processes and concepts of the present disclosure, the pH microenvironment present both on and within a medical device, which can impact thrombus formation kinetics, may be altered such that thrombus formation is slowed or avoided at or close to the surfaces of the device, thus preventing coagulation on the device.

Any surface of a medical device that may come into contact with blood, blood components, or other bodily fluids which may form a coagulum may be treated in accordance with the present disclosure. Thus, the medical devices of the present disclosure may be any medical device which may contact blood or constituents thereof. The medical device may be an implanted device such as catheters, stents, defibrillators, guidewires, and grafts, or may be an external device, such as blood transfer devices like hemodialyzers and other dialysis and plasmapheresis devices, blood oxygenators, and extracorporeal circuits. Any device which permits the flow of blood or blood constituents over a surface or through the interior of the device may benefit from the processes and treatments of the present disclosure.

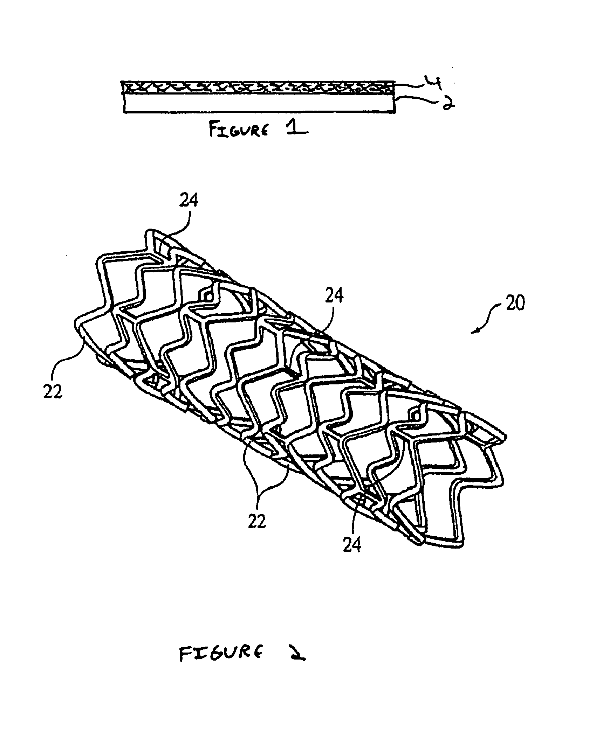

The effects obtained with the pH microenvironment according to the present disclosure may be localized and transient. As depicted in FIG. 1, surface 2 of a medical device of the present disclosure may have a pH microenvironment 4 near the surface 2 of the medical device. Thus, the effects obtained within the microenvironment 4 adjacent the surface 2 will not impact the bulk of the medical device nor the bulk pH of blood in the area.

Referring to FIG. 2, a medical device subjected to the treatments of the present disclosure to control the pH microenvironment near its surface may include a stent 20. Stent 20 can have the form of a tubular member defined by a plurality of struts. The struts can include a plurality of bands 22 and a plurality of connectors 24 that extend between and connect adjacent bands. During use, bands 22 can be expanded from an initial, small diameter to a larger diameter to contact the stent 20 against a wall of a vessel, thereby maintaining the patency of the vessel. Connectors 24 can provide stent 20 with flexibility and conformability that allow the stent to adapt to the contours of the vessel.

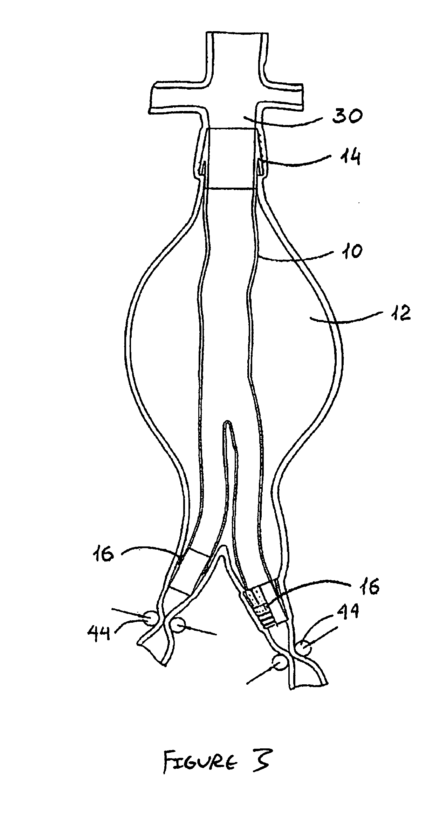

Referring to FIG. 3, in other embodiments a vascular graft 10 may be subjected to the treatments of the present disclosure to control the pH microenvironment near its surface. FIG. 3 illustrates a cross sectional view of a bifurcated graft 10 inserted within an aortic aneurysm. A bifurcated graft 10 is inserted within an aneurysm 12 in a blood vessel. In embodiments, three ends of bifurcated graft 10 are provided with concentric docking heads, a first docking head 14 at the proximal end and two docking heads 16 at the distal ends of the graft. Docking heads 14 and 16 are adapted to couple the graft to the vessel, in embodiments without suturing, and provide the surgeon with the ability to rapidly connect the graft to the aneurysm.

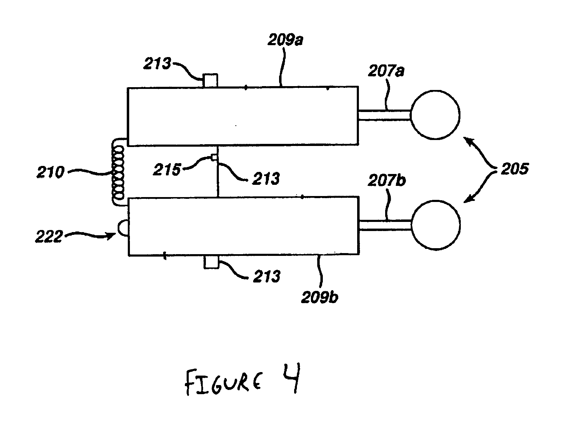

Referring to FIG. 4, in other embodiments an internal defibrillator may be subjected to the treatments of the present disclosure to control the pH microenvironment near its surface. The electrodes (205) are respectively attached to one end a pair of paddles (207a, 207b). The paddles are connected to the respective left and right handles (209a, 209b). The left and right handles are electrically connected by at least one wire (210). It should be noted that any flexible conductor and/or flex board could provide a conduction path. Optionally, the handles (209a, 209b) can be arranged on a slidable track (213) which allows the electrodes (209a, 209b) to be spaced according to need. The track may have a locking mechanism (215) to hold the handles (and thus the electrodes) at the desired distance from each other. This locking mechanism could be a latch, or a wingnut and a bolt that can travel within a slot cut into the track, a hook, or any known type of lock device that a user can both lock and/or release quickly.

As discussed above, charged polymers assist in preventing thrombus formation on various medical devices. In one embodiment, a charged polymer is integrally formed with a medical device to prevent thrombus formation on a surface thereof. In another embodiment, a blood contacting surface of a medical device is coated with a charged polymer to assist in preventing thrombus formation of blood passing thereover or therethrough, in the case of medical devices including a lumen or other channel or passage for blood flow. The medical device of the present disclosure may thus avoid thrombus related failures of medical devices that may otherwise occur due to thrombus formation on or in such medical devices.

Bioactive agents may be added to the medical devices of the present disclose to provide specific biological or therapeutic properties thereto. Any product which may enhance tissue repair, limit the risk of sepsis, and modulate the mechanical properties of the medical device may be added during the preparation of the device or may be coated on the device.

Moreover, the medical device may also be used for delivery of one or more bioactive agents. The bioactive agents may be incorporated into the medical device during formation of the device, such as by free suspension, liposomal delivery, microspheres, etc., or by coating a surface of the medical device, or portion thereof, such as by polymer coating, dry coating, freeze drying, applying to a mesh surface, ionically, covalently, or affinity binding. In other embodiments, bioactive agents may be coated onto a surface or a portion of a surface of the medical device for release of the bioactive agent.

A bioactive agent as used herein is used in the broadest sense and includes any substance or mixture of substances that have clinical use. Consequently, bioactive agents may or may not have pharmacological activity per se, e.g., a dye. Alternatively a bioactive agent could be any agent that provides a therapeutic or prophylactic effect; a compound that affects or participates in tissue growth, cell growth, and/or cell differentiation; an anti-adhesive compound; a compound that may be able to invoke a biological action such as an immune response; or could play any other role in one or more biological processes. A variety of bioactive agents may be incorporated into the medical device.

Examples of classes of bioactive agents which may be utilized in accordance with the present disclosure, include, for example anti-adhesives; antimicrobials; analgesics; antipyretics; anesthetics; antiepileptics; antihistamines; anti-inflammatories; anti-thrombogenic; cardiovascular drugs; diagnostic agents; sympathomimetics; cholinomimetics; antimuscarinics; antispasmodics; hormones; growth factors; muscle relaxants; adrenergic neuron blockers; antineoplastics; immunogenic agents; immunosuppressants; gastrointestinal drugs; diuretics; steroids; lipids; lipopolysaccharides; polysaccharides; platelet activating drugs; clotting factors; and enzymes. It is also intended that combinations of bioactive agents may be used.

Other bioactive agents, which may be included as a bioactive agent include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; antihistamines; and immunological agents.

Other examples of suitable bioactive agents, which may be included in the medical device include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids, such as antisense molecules, DNA, RNA, RNAi; oligonucleotides; polynucleotides; and ribozymes.

It should be understood that various combinations of medical devices and pH modulating materials may be used in accordance with the present disclosure. For example, any medical device may be combined with any pH modulating material as described above, dependent upon the pH microenvironment desired.

While several embodiments of the disclosure have been described, it is not intended that the disclosure be limited thereto, as it is intended that the disclosure be as broad in scope as the art will allow and that the specification be read likewise. Therefore, the above description should not be construed as limiting, but merely as exemplifications of embodiments of the present disclosure. Various modifications and variations of the medical device, as well as methods of forming the medical device and materials for modifying the pH of the surface and thus the microenvironment, will be apparent to those skilled in the art from the foregoing detailed description. Such modifications and variations are intended to come within the scope and spirit of the claims appended hereto.

The invention may be described by reference to the following numbered paragraphs:-

A medical device comprising: at least one substrate possessing a surface; and a functionality on at least a portion of the surface, wherein the functionality modulates a pH of a microenvironment near the surface of the medical device.

The medical device of paragraph **[0052],** wherein the functionality on at least a portion of the surface is affected by a charged polymer.

The medical device of paragraph **[0052],** wherein the medical device is implantable.

The medical device of paragraph **[0054],** wherein the medical device is selected from the group consisting of catheters, stents, defibrillators, guidewires, and grafts.

The medical device of paragraph **[0052],** wherein the medical device is external to a body of a patient.

The medical device of paragraph **[0056],** wherein the medical device is selected from the group consisting of blood transfer devices, dialysis devices, plasmapheresis devices, blood oxygenators, and extracorporeal circuits.

The medical device of paragraph **[0052],** wherein the surface of the medical device comprises a lumen of the medical device.

The medical device of paragraph **[0052],** wherein the surface of the medical device is a tissue facing surface.

The medical device of paragraph **[0052],** wherein the surface of the medical device is a blood contacting surface.

The medical device of paragraph **[0052],** wherein the charged polymer comprises a coating on at least a portion of the surface of the medical device.

The medical device of paragraph **[0052],** wherein the charged polymer possesses a negative charge.

The medical device of paragraph **[0052],** wherein the charged polymer possesses a positive charge.

The medical device of paragraph **[0052],** wherein the charged polymer is selected from the group consisting of 2-hydroxyethyl methacrylate, 2-acrylamido-2-methylpropane sulfonic acid, 3-methacryloylaminopropyl-trimethyl ammonium chloride, and combinations thereof.

The medical device of paragraph **[0052],** wherein the charged polymer comprises a positively charged copolymer of 3-methacryloylaminopropyl-trimethyl ammonium chloride with 2-hydroxyethyl methacrylate.

The medical device of paragraph **[0052],** wherein the charged polymer comprises a negatively charged copolymer of 2-acrylamido-2-methylpropane sulfonic acid with 2-hydroxyethyl methacrylate.

The medical device of paragraph **[0052],** wherein the charged polymer is selected from the group consisting of hydroxypropyl methylcellulose, methacrylic acid copolymers, and combinations thereof, in combination with an acid.

The medical device of paragraph **[0067],** wherein the charged polymer comprises a copolymer of methacrylic acid with ethyl acrylate.

The medical device of paragraph **[0067],** wherein the acid is selected from the group consisting of citric acid, fumaric acid, succinic acid, malic acid, and combinations thereof, present in an amount of from about 0.1 percent by weight to about 10 percent by weight of the copolymer.

A medical device comprising: at least one substrate possessing a surface; and a functionality on at least a portion of the surface, wherein the functionality modulates a pH of a microenvironment near the surface of the medical device to prevent thrombus formation.

The medical device of paragraph **[0070],** wherein the functionality on at least a portion of the surface is affected by a charged polymer.

## Claims

1. A medical device comprising:
at least one substrate possessing a surface; and
a functionality on at least a portion of the surface,
wherein the functionality modulates a pH of a microenvironment near the surface of the medical device.

2. The medical device of claim 1, wherein the functionality on at least a portion of the surface is affected by a charged polymer.

3. The medical device of claim 1 or claim 2, wherein the medical device is implantable.

4. The medical device of claim 3, wherein the medical device is selected from the group consisting of catheters, stents, defibrillators, guidewires, and grafts.

5. The medical device of any preceding claim, wherein the medical device is external to a body of a patient, preferably wherein the medical device is selected from the group consisting of blood transfer devices, dialysis devices, plasmapheresis devices, blood oxygenators, and extracorporeal circuits.

6. The medical device of any preceding claim, wherein the surface of the medical device comprises a lumen of the medical device.

7. The medical device of any of claim 1 to 5, wherein the surface of the medical device is a tissue facing surface.

8. The medical device of any preceding claim, wherein the surface of the medical device is a blood contacting surface.

9. The medical device of any preceding claim, wherein the charged polymer comprises a coating on at least a portion of the surface of the medical device.

10. The medical device of any preceding claim, wherein the charged polymer possesses a negative charge; or wherein the charged polymer possesses a positive charge.

11. The medical device of claim 10, wherein the charged polymer is selected from the group consisting of 2-hydroxyethyl methacrylate, 2-acrylamido-2-methylpropane sulfonic acid, 3-methacryloylaminopropyl-trimethyl ammonium chloride, and combinations thereof.

12. The medical device of claim 11, wherein the charged polymer comprises a positively charged copolymer of 3-methacryloylaminopropyl-trimethyl ammonium chloride with 2-hydroxyethyl methacrylate; or wherein the charged polymer comprises a negatively charged copolymer of 2-acrylamido-2-methylpropane sulfonic acid with 2-hydroxyethyl methacrylate.

13. The medical device of claim 10, wherein the charged polymer is selected from the group consisting of hydroxypropyl methylcellulose, methacrylic acid copolymers, and combinations thereof, in combination with an acid, preferably wherein the acid is selected from the group consisting of citric acid, fumaric acid, succinic acid, malic acid, and combinations thereof, present in an amount of from about 0.1 percent by weight to about 10 percent by weight of the copolymer.

14. The medical device of claim 13, wherein the charged polymer comprises a copolymer of methacrylic acid with ethyl acrylate.

15. A medical device comprising:
at least one substrate possessing a surface; and
a functionality on at least a portion of the surface,
wherein the functionality modulates a pH of a microenvironment near the surface of the medical device to prevent thrombus formation.

16. The medical device of claim 15, wherein the functionality on at least a portion of the surface is affected by a charged polymer.
